(19) Europäisches Patentamt
European Patent Office
Office européen des brevets

(11) Publication number: 0 170 751 B1

# (12) EUROPEAN PATENT SPECIFICATION

(45) Date of publication of patent specification: 15.03.89

(51) Int. Cl.$^4$: C 01 B 33/28, B 01 J 29/28

(21) Application number: 84305485.9

(22) Date of filing: 10.08.84

## (54) Novel crystalline aluminosilicate, process for the preparation thereof, and process for the conversion of organic materials using the same.

(43) Date of publication of application:
12.02.86 Bulletin 86/07

(45) Publication of the grant of the patent:
15.03.89 Bulletin 89/11

(84) Designated Contracting States:
DE FR GB NL

(56) References cited:
EP-A-0 087 720
EP-A-0 094 693
EP-A-0 098 641
US-A-4 257 885

PATENTS ABSTRACTS OF JAPAN, vol. 7, no. 131 (C169)1276r, 8th June 1983; & JP - A - 58 45111 (TOA NENRYO KOGYO K.K.) 16-03-1983

PATENTS ABSTRACTS OF JAPAN, vol. 8, no. 41 (C-211)1478r, 22nd February 1984; & JP - A - 58 199 714 (TOA NENRYO KOGYO K.K.) 21-11-1983

(73) Proprietor: TOA NENRYO KOGYO KABUSHIKI KAISHA
1-1 Hitotsubashi, 1-Chome Chiyoda-Ku
Tokyo 100 (JP)

(72) Inventor: Sakurada, Satoshi
1502-5 Oaza -Omaki Urawa-shi
Saitama-ken (JP)
Inventor: Tagaya, Nobuaki
2735-4, Oaza-Kasahata
Kawagoe-shi Saitama-ken (JP)
Inventor: Miura, Tadashi
1160-3, Oaza-Ogimachiya
Iruma-shi Saitama-ken (JP)
Inventor: Maeshima, Tsugio
1428-34 Oaza-Suneori Tsurugashima-cho
Iruma-gun Saitama-ken (JP)
Inventor: Hashimoto, Takao
19025 Oaza-Kamekubo Ohi-machi
Iruma-gun Saitama-ken (JP)

(74) Representative: Dixon, Donald Cossar et al
Gee & Co. Chancery House Chancery Lane
London WC2A 1QU (GB)

EP 0 170 751 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel crystalline aluminosilicate, a process for the conversion of an organic material using said crystalline aluminosilicate, and a process for the preparation of a novel crystalline sodium aluminosilicate. More particularly, the present invention relates to a novel crystalline aluminosilicate having a characteristic distribution of Si and Al, in the external surface, a process for the conversion of an organic material utilizing the molecular shape selectivity and catalytic ability of said crystalline aluminosilicate, and a process for the preparation of a novel crystalline sodium aluminosilicate.

Crystalline aluminosilicates, generally known as crystalline zeolites, are aluminosilicate hydrates. Their crystalline structure, whether they are natural or synthetic are based on a three-dimensional framework of an $SiO_4$ tetrahedron, wherein four oxygen atoms centering around silicon (Si) are positioned at vertices, and of an $AlO_4$ tetrahedron which is formed by replacing silicon atom of the $SiO_4$ with aluminum (Al).

It is known that these $SiO_4$ and $AlO_4$ tetrahedra form fundamental units of 4, 5, 6, 8, or 12-membered rings formed by the interconnection of 4, 5, 6, 8, or 12 of them and double rings formed by superposing these 4, 5, 6, 8, and 12-membered rings on each other, and that the framework structure of a crystalline aluminosilicate is determined by the interconnection of these units. There are specified cavities in the framework structure determined by the above interconnection. An entrance of this cavity structure is an opening formed with 6, 8, 10, and 12-membered rings. Openings, described above, have a unified diameter so that only molecules smaller than a predetermined size can be adsorbed, and larger ones, which cannot enter through the openings are not adsorbed. This action of the crystalline aluminosilicate is known as the "molecular sieve", which has been utilized as an adsorbent, a catalyst, or a catalyst carrier for chemical reactions in various chemical processes.

In recent years, intensive studies have been made in various fields of chemical reactions for the combined use of crystalline aluminosilicates as the above-mentioned molecular sieve and as catalyst. This is the application as a so-called molecular shape selective reaction catalyst, as classified from the viewpoint of catalyst function by S. M. Csicsery into the following three groups: (1) those characterized in that only specified reactants can approach to an active site; (2) those characterized in that, after the reaction at the active site, only the substances of the specified shape can leave from the site of reaction; and (3) those characterized in that, in a bimolecular reaction, each molecule can freely approach to or leave from the reaction site but they cannot react because the transition state is too large ("Zeolite Chemistry and Catalysis" ACS Monograph 171, ACS, Washington D.C., 1976, p. 680).

The above classification is based on a consideration that the reaction occurs only in the cavity of crystalline aluminosilicate. The catalytic reaction on or near the active site of external surface of crystal is low in reaction selectivity, unlike the catalytic reaction described above, because all kinds of reaction are possible and reactions occur freely beginning with the reaction having small activation energy.

In order to suppress these non-selective reactions on or around "the external surface of crystals" (hereinafter referred to simply as "the crystal surface"), methods of burying active sites by covering the crystal surface with a compound, or controlling the solid acid acidity of the active sites by using alkaline substances or those with different solid acid acidity. Addition of silicon, phosphorus, or magnesium compounds have been suggested.

On the other hand, a method of controlling the size of the crystals is known, thereby controlling the ratio of the number of the active sites having molecular shape selectivity in the crystals and that of the active sites not having molecular shape selectivity on or around the crystal surface. For example, when the crystals are large, the ratio of the number of the active sites in the crystals is relatively increased, thereby the molecular shape selectivity increases. However, this results in lower reactivity in general, because of the relative limitation in approach and/or contact of the reactants to the active sites. On the other hand, when crystals are small, the reactivity is elevated because of relative increase in the opportunity of approach and/or contact of the reactants to the active sites, although the molecular shape selectivity is lowered as the result of relative increase of the number of the active sites on or around the crystal surface.

The electric charge of the tetrahedral crystalline sodium aluminosilicate, containing aluminum is balanced in an equilibrated state by incorporating sodium cation in the crystal. This cation is well known to act as solid acid catalyst when it is ion-exchanged into a hydrogen- or metallic ion-exchanged type in various methods.

It is understood therefore that the places where Al atoms are present are intimately related, directly or indirectly, to those where active sites are present. It is also fully expected that the solid acid strength of an adjacent atom (silicon) bonded to the Al atom with an interposed oxygen atom is strongly influenced by the neighboring atom (silicon or another aluminum) to which it is bonded with another interposed oxygen atom.

It was elucidated by the so-called Loewenstein's rule (W. Loewenstein, Am. Miner. *39*, 92 (1954)) that the bond of an aluminum atom to another aluminum atom with interposed oxygen can not exist.

It is understood from the above that if the distribution of Al atoms in a crystalline sodium aluminosilicate can be controlled, the solid acid strength itself or its distribution in the crystalline sodium aluminosilicate as catalyst can be controlled freely. Moreover, if the distribution of Al atoms in crystals can be controlled, the active sites can be concentrated to the inside of crystals or contrarily concentrated on or around the external surface of crystals, whereby the same effect as that, attained when the solid acid acidity

is controlled by the coverage with compounds, is expected. The amount of the compounds to be incorporated for modification is also expected to be reduced.

Although structures of crystalline aluminosilicates have been analyzed with X-ray, most of them only show a so-called framework structure, and there are few works in which exact positions of the silicon and aluminum atoms were determined. It is because the X-ray scattering ability of a silicon and an aluminum atoms are very similar, and because relatively large single crystals are hardly obtained.

However, since the surface elemental analysis of crystals in the order of micron has become possible by the recent development in surface analyzing technique, it has become possible to discuss about the elemental distribution in crystals.

As described above, the electric charge of the aluminum-containing tetrahedral crystalline aluminosilicate is balanced in an equilibrated state by cations incorporated in the crystal. In natural crystalline aluminosilicates, they are cations belonging to Group I or II metal of the periodic table, preferably those of sodium, potassium, calcium, magnesium, or strontium. These metallic cations are also used in synthetic crystalline aluminosilicates, but recently the use of organic nitrogen cations, for example, quaternary alkylammonium ions such as tetraalkylammonium ions, are suggested. These nitrogen-containing organic compounds were considered essential as an alkali source for the synthesis of crystalline aluminosilicates having a high silica/alumina ratio.

However, the use of nitrogen-containing organic compounds is disadvantageous in high material cost, as well as complicated for the preparation, because they must be removed by firing at high temperatures when the obtained synthetic aluminosilicate is used as catalyst.

Moreover, there have been problems in safety of operation in the conventional process wherein the above-described tetraalkylammonium compounds or organic amine compounds such as $C_2$ to $C_{10}$ primary amines are used. Because these organic compounds are dangerous themselves as well as decomposed products of them. Therefore they may bring out various dangers in steps of synthesis, drying, and firing.

The present inventors, as will be described briefly below, overcame these problems and provided a process for the preparation of a crystalline aluminosilicate from an aqueous reaction mixture substantially consisting of only inorganic reaction materials (Japanese Patent Application No. 143396/1981 filed on September 11, 1981).

The inventors also elucidated that the obtained crystalline aluminosilicate had a characteristic crystalline structure as shown by the X-ray diffraction pattern.

The above application was related to a crystalline aluminosilicate having a chemical composition expressed in terms of oxide molar ratios:

$$0.8\text{—}1.5\ M_2/nO \cdot Al_2O_3 \cdot 10\text{—}100\ SiO_2 \cdot zH_2O,$$

wherein M stands for a metallic cation, n for the valence of said metallic cation, and z for 0 to 40, and having a powder X-ray diffraction pattern showing at least the interplanar spacings, namely, shown in Table 1.

TABLE 1

| Interplanar spacing, d(Å) | Relative intensity ($I/I_o$) |
|---|---|
| 11.2±0.2 | S. |
| 10.1±0.2 | S. |
| 7.5±0.15 | W. |
| 6.03±0.1 | M. |
| 4.26±0.07 | M. |
| 3.86±0.05 | V.S. |
| 3.82±0.05 | S. |
| 3.76±0.05 | S. |
| 3.72±0.05 | S. |
| 3.64±0.05 | S. |

The aluminosilicate having the crystalline structure characterized by the above X-ray diffraction pattern, which has not been found in literature, was named TSZ.

The above values are obtained by the measurement with an X-ray diffraction device manufactured by Rigaku Denki K.K. (Geigerflex RAD-rA) in the ordinary method. Irradiation was carried out with K-α doublet of copper. The relative intensity and the interplanar spacings (d) expressed in terms of angstrom (Å) corresponding to the recorded lines were determined from the height and position of the peak read from the chart as a function of 2θ (θ=Bragg angle) by using a scintillation counter provided with a strip chart pen recorder. The relative intensity in Table 1 is evaluated as very strong by "V.S.", strong by "S", medium by "M", weak by "W", and very weak by "V.W.". As will be described below, the crystalline aluminosilicate of the above application is characterized by the X-ray diffraction pattern obtained by the ordinary powder X-ray diffraction method. More particularly, the characteristics which distinguish the crystalline aluminosilicate of the above applications most remarkably from the conventional crystalline zeolites in

crystalline structure are that the diffraction line of $2\theta=14.7°$ (d=6.03 A) is singlet and those of $2\theta=23°$ (d=3.86 Å) and of $2\theta=23.3°$ (d=3.82 Å) are clearly split.

The crystalline aluminosilicate of the above application was also subjected to powder X-ray diffraction analysis different from the ordinary method, by which $2\theta$ ($\theta$=Bragg angle) was measured with remarkably high accuracy. The analysis of the result showed that the crystalline aluminosilicate of the above application (TSZ) belonged to a monoclinic system crystallographically. For example, the TSZ having a composition of

$$1.02Na_2O \cdot Al_2O_3 \cdot 26.2\ SiO_2 \cdot 12.2\ H_2O,$$

the product of Example 7 in the Japanese Patent Application No. 1433396/1981, shows the following monoclinic lattice constant: a=20.159 (±0.004) Å, b=19.982 (±0.006) Å, c=13.405 (±0.005) Å, and α=90.51° (±0.03°). The observed and calculated values and Miller indices of the interplanar spacing of this typical TSZ are shown in Table 2. The interplanar spacing of this characteristic X-ray diffraction pattern was not much influenced by exchanging the substituting cations of the synthetic aluminosilicate, especially by exchanging to hydrogen ion, and also not influenced by change of the ratio $SiO_2/Al_2O_3$.

The present inventors have arrived at the present invention as a result of further intensive studies based on the above-described previous invention, finding that (1) a crystalline aluminosilicate having a novel surface structure possessing remarkable molecular shape selectivity and catalytic ability, in which the distribution of aluminum atoms in the crystal is controlled and the solid acidity distribution is approximately homogeneous can be provided, that (2) such crystalline aluminosilicate can be prepared from substantially inorganic reaction materials, and that (3) such crystalline aluminosilicate brings about good results in selective catalytic reactions of hydrocarbons.

TABLE 2

| Interplanar spacing | | Miller index (hkl) | Interplanar spacing | | Miller index (hkl) |
|---|---|---|---|---|---|
| Observed | Calculated | | Observed | Calculated | |
| 11.18 (Å) | 11.16 (Å) | 101 | 4.372 (Å) | 4.369 (Å) | $01\bar{3}$ |
| 10.09 | 10.08 | 200 | 4.272 | 4.271 | $42\bar{1}$ |
| 9.978 | 9.990 | 020 | 4.097 | 4.093 | $02\bar{3}$ |
| 9.765 | 9.775 | $11\bar{1}$ | 4.018 | 4.019 | 430 |
| 9.028 | 8.999 | 210 | 3.863 | 3.861 | 501 |
| 7.465 | 7.458 | 211 | 3.825 | 3.820 | 0.51 |
| 7.095 | 7.096 | 220 | 3.757 | 3.754 | 151 |
| 6.720 | 6.703 | 002 | 3.727 | 3.726 | $03\bar{3}$ |
| 6.383 | 6.372 | $01\bar{2}$ | 3.654 | 3.653 | 313 |
| 6.013 | 6.007 | 301 | 3.605 | 3.604 | $52\bar{1}$ |
| 5.719 | 5.738 | $13\bar{1}$ | 3.490 | 3.495 | $32\bar{3}$, $23\bar{3}$ |
| 5.587 | 5.589 | $02\bar{2}$ | 3.450 | 3.449 | 530 |
| 5.381 | 5.386 | $21\bar{2}$, $12\bar{2}$ | 3.361 | 3.360 | 600 |
| 5.150 | 5.147 | $23\bar{1}$ | 3.320 | 3.321 | 351 |
| 5.039 | 5.040 | 400 | 3.259 | 3.259 | $33\bar{3}$ |
| 4.990 | 4.995 | 040 | 3.056 | 3.055 | $45\bar{1}$ |
| 4.624 | 4.624 | $31\bar{2}$ | 2.988 | 2.996 | $43\bar{3}$ |

The first object of the present invention is, therefore to provide a crystalline aluminosilicate having a characteristic crystalline structure in which the distribution of aluminum atoms in the crystal is controlled.

The second object of the present invention is to provide a crystalline aluminosilicate having a high silica/alumina ratio and excellent catalytic activity.

The third object of the present invention is to provide a crystalline aluminosilicate having a characteristic crystalline structure exhibiting excellent activities in selective cracking of linear hydrocarbons and conversion of aromatics.

The fourth object of the present invention is to provide a process for the preparation of a crystalline sodium aluminosilicate having a characteristic crystalline structure, in the crystal of which the distribution of aluminum atoms is controlled, from an aqueous reaction mixture substantially consisting of inorganic reaction materials such as silicon, aluminum, alkali metal compounds, and water.

The fifth object of the present invention is to provide a process for the preparation of a crystalline sodium aluminosilicate, having such

$$Na_2O—Al_2O_3—SiO_2—H_2O$$

composition, that enables the reduction in production cost based on an easy and simple process for the production, without any heat-treating process of the product, which has been necessary in the conventional preparation of synthetic aluminosilicates.

Further, the sixth object of the present invention is to provide a conversion process for organic materials using as catalyst a novel crystalline aluminosilicate wherein the distribution of aluminum is controlled.

Thus, the present invention relates to a novel crystalline aluminosilicate characterized in that the silicon/aluminum atomic ratio on the surface of the crystal is not smaller than average value of the silicon/aluminum atomic ratio in whole crystal, a process for the conversion of organic materials using said crystalline aluminosilicate and a process for the preparation of a novel crystalline sodium aluminosilicate.

The crystalline aluminosilicate of the present invention (TSZ-III) is of great value for its catalytic activity in a variety of organic reactions including hydrogenation-isomerization of normal paraffins, conversion of alcohols into hydrocarbons, alkylation of aromatics with alcohol and olefin, disproportionation or transalkylation between aromatic compounds and so on, especially showing remarkable effects in the cracking of hydrocarbons. Moreover, according to the present invention, such useful crystalline aluminosilicate (TSZ-III) can be prepared extremely easily and economically.

Embodiments of the present invention will now be described, by way of example, with reference to the accompanying drawings in which:

Figures 1 and 2 are graphical illustrations of the relationship between the amount of the desorbed pyridine and temperature.

In Figure 1, the solid line shows the results of the gas-chromatographic measurement of the sample of the present invention obtained in Example 4, and the broken line shows that of the sample obtained in Comparative Example 5.

In Figure 2, the solid line shows the results of the gas-chromatographic measurement of the sample of the present invention obtained in Example 1, and the broken line shows that of the sample obtained in Comparative Example 1.

In the present invention the distribution of atoms on or around "the external surface of the crystal" (abbreviated as "the crystal surface") of the crystalline aluminosilicate was measured by X-ray photoelectron spectroscopy.

It is necessary to reduce the number of the active sites having no molecular shape selectivity for the elevation of reaction selectivity in selective cracking of linear hydrocarbons, or in isomerization, alkylation and disproportionation of aromatic hydrocarbons. It is important for this purpose to rélatively decrease the number of aluminum atoms which tend to form a framework on or around the crystal surface. When the sample is irradiated with X-ray, a photoelectron is released from the sample. Since the electron interacts with substances strongly, its mean free path is small. Therefore the electrons released from the depth of the sample are scattered inside the sample and lose their energy, then they cannot go to the surface. In that case, the depths from which electrons can escape to the surface (escape depth) depends on the sort of element and the kinetic energy of the electron. Aluminum has an escape depth of 20 Å in average (kinetic energy: 1,500 ev) and silicon has about 20 to 40 Å (kinetic energy: 554~1,178 ev).

On the other hand, the depth of the crystal is not recognized where the active sites, which have not high molecular shape selectivity, is able to exist. However, it is assumed that the active sites which are present at 20 Å deep or more from the top surface are provided with sufficient molecular shape selectivity, because the size of the crystal is usually within the range of 1,000 Å to several tens of thousand Å and the size of the molecules taking part in the reaction is 10 Å or less. In the present invention, therefore, X-ray photoelectron spectroscopy is the most suitable method of analysis. It is preferred for the measurement by X-ray photoelectron spectroscopy to use aluminum-Kα ray as X-ray source with an X-ray output of 10 kv-20 mA. The measured silicon/aluminum atomic ratio in this way is defined as "silicon/aluminum atomic ratio on the external surface of a crystal".

On the other hand, the silicon/aluminum atomic ratio obtained by an ordinary chemical analysis such

as atomic absorption is defined as "average value of the silicon/aluminum atomic ratio in whole crystal" (abbreviated as "average silicon/aluminum atomic ratio").

The aluminum distribution inside the crystal is able to be estimated by determining an adsorption/desorption curve of pyridine, from which the solid acid strength distribution of whole crystal is determined. Because, both of the silicon/aluminum arrangement with a bridged oxygen atom inside the crystal and the arrangement of silicon/aluminum atoms on the crystal surface relate to the strength distribution of solid acid.

Moreover, it is generally known that pyridinium ions or coordinated pyridine are formed by adsorbing pyridine on the solid acid sites, but this pyridine is desorbed as temperature is raised, and the desorption temperature and solid acid strength is intimately interrelated.

In the present invention, pyridine is sufficiently adsorbed at 300°C by H (hydrogen-type)-TSZ-III (TSZ-III will be defined later) obtained by the ion-exchange of crystalline aluminosilicate with ammonium chloride and/or mineral acid and successive firing at about 500°C. Then H-TSZ-III is maintained in a nitrogen stream at 300°C so that the physically adsorbed pyridine is removed off. Thereafter the temperature is raised at a predetermined rate to quantitatively determine the desorbed pyridine by gas chromatography or other ordinary methods of measurement, whereby the amount and the strength distribution of the solid acid are determined from the height, shape, or position of the peak obtained.

The process for the preparation of crystalline sodium aluminosilicate of the present invention will now be described.

The crystalline aluminosilicate of the present invention, having a characteristic distribution of silicon and aluminum atoms in its crystal (hereinafter referred to as TSZ-III), is usually prepared as follows.

An aqueous reaction mixture substantially consisting of inorganic reaction materials is prepared by using $SiO_2$ as silicon source and $Al_2O_3$ as aluminum source, within a certain range of ratio, and some appropriate alkali source and water. These components are mixed in predetermined range, then the reaction mixture is heated at a crystallization temperature until the crystals are formed.

The composition of the aqueous reaction mixture for the preparation of the crystalline sodium aluminosilicate of the present invention is as follows:

| | |
|---|---|
| $SiO_2/Al_2O_3$ | 10~100 |
| $Na_2O/SiO_2$ | 0.03~0.5 |
| $H_2O/SiO_2$ | 10~300 |
| $Cl^-/SiO_2$ | 0.1~10 |

still preferably:

| | |
|---|---|
| $SiO_2/Al_2O_3$ | 20~80 |
| $Na_2O/SiO_2$ | 0.05~0.3 |
| $H_2O/SiO_2$ | 20~200 |
| $Cl^-/SiO_2$ | 0.1~5 |

The preparation of this aqueous reaction mixture, and of the subsequent aqueous reaction gel mixture, are especially important for the preparation of TSZ-III.

Generally, there are no essential difference between the preparation of crystalline sodium aluminosilicate using inorganic reaction materials as in the present invention and the preparation of crystalline sodium aluminosilicate using organic nitrogen cations instead of cations of a Group I or II metal. In either case, an aqueous reaction gel mixture can be obtained at the same time when the acidic solution of silicon and the alkaline solution of aluminum are mixed together. In another method, the prepared homogeneous solution of silicon and aluminum can be gelated with an acid or alkali. Further, the obtained gel can be converted into xerogel and then reconverted into aqueous reaction gel mixture.

Among these various methods of gelation, the most preferred one for the preparation of TSZ-III (in which the distributions of silicon and aluminum in the crystal must be controlled) is a method of preparing a homogeneous solution of silicon and aluminum compounds firstly and then gelating the solution. Any combination of compounds can be used so long as a homogeneous solution of silicon and aluminum is obtained. For example, active alumina, γ-alumina, alumina trihydrate, sodium aluminate, and chloride, nitrate, and sulfate of aluminum are used as aluminum source, and sodium silicate, silica gel, silicic acid, aqueous colloidal silica gel, dissolved silica, powdered silica, or amorphous silica as silica source. The most preferable sources are an aqueous sodium aluminate solution as aluminum compound source and aqueous solutions of various sodium silicates or colloidal silica as silica compound source, considering properties of the obtained crystals, economy, scale merit of industrialization, and so on.

Thus, a homogeneous mixture of silicon and aluminum is obtained in the form of an alkaline solution, and an aqueous reaction gel mixture is obtained by neutralizing this alkaline solution with an acid solution. It is preferred to use a so-called mineral acid, more preferably hydrochloric, sulfuric, or nitric acid for the neutralization. Hydrochloric acid is the most desirable because sodium chloride, formed by the reaction with excess sodium hydroxide, serves as a mineralizer. The use of the mineralizer is desirable as will be described below for further improving the crystallizability to suppress the formation of amorphous sodium aluminosilicate.

A solution of metal alkoxide can also be used to obtain a homogeneous solution of silicon and aluminum compounds.

The alkoxide compounds of silicon include, for example, methyl orthosilicate (tetramethyl orthosilicate), or ethyl orthosilicate (tetraethyl orthosilicate).

The typical alkoxide compounds of aluminum include aluminum isopropoxide.

A homogeneous solution of silicon and aluminum compounds can be prepared easily by mixing the metal alkoxide solution. A homogeneous gel can be obtained easily by the hydrolysis of the obtained solution in the presence of an acid or an alkali. An aqueous reaction gel mixture can be also obtained from a glassy compound formed by moderate hydrolysis of above obtained homogeneous solution of silicon and aluminum compounds. In this case also, a mineralizer such as sodium chloride is effective for improving the crystallizability of the obtained crystalline sodium aluminosilicate.

The following two mechanisms are assumed as a mechanism of forming crystalline aluminosilicate from an aqueous reaction gel mixture.

The first mechanism include the depolymerization and dissolution of gels in hydrothermal reaction so that separately formed crystalline nuclei grow gradually. Since the crytalline nuclei are formed centering around a cation, it is assumed that the organic cation establishes a circumstance in which crystalline nuclei are easily formed according to its action as "template". Since the pH value of the solution is relatively higher in the presence of an organic cation than in the presence of an inorganic cation, the nuclei are considered to be formed with a cation serving as "template" immediately, after the formation of a hydrated silicate ion $(H_2O)Si(OH)_5^-$ by the action of the organic cation. The action of the hydroxide ion is important in this depolymerization gels.

It is considered that, in this mechanism, the deposition and subsequent crystallization of silicon compounds occur continuously around the nuclei (which contain a relatively large amount of silicon) in the presence of the organic cation. If these nuclei are assumed to grow infinitely, it is not unreasonable that, as has been suggested, crystalline silicate consisting of silicon alone exists. Actually, it is known that the molar ratio of silica/alumina can not be reduced to 20 or less, while it is possible to control freely the molar ratio of silica/alumina of the obtained crystalline aluminosilicate within the range from 20 to several thousands, when organic ion such as tetrapropyl ammonium salt is added in the reaction mixture.

The second mechanism include the formation of an aluminosilicate framework without dissolution of the gel. In this case, it is also considered that the nuclei grow in the gel phase and re-arrange from the gel phase into solid. In this case, of course, the silicon-aluminum arrangement in the aqueous reaction gel mixture is considered to affect greatly the silicon-aluminum arrangement of the obtained crystalline aluminosilicate. The assumption of the second formation mechanism is advantageously supported by the fact that, if the molar ratio of silica/alumina in the aqueous reaction gel mixture is increased to 100 or more with the purpose of elevating the molar ratio of silica/alumina in the obtained crystalline sodium aluminosilicate, the rearrangement becomes difficult and silicon oxide crystals are formed.

According to the second formation mechanism, it is understood that the arrangement of silicon/alumina in the gel particles must be controlled in order to control that in the crystals.

Anyway, distribution of silicon and aluminum in the gel particles existing in a gel mixture should be homogeneous because the aqueous reaction gel mixture is obtained from a homogeneous solution. This homogeneous distribution in the gel particles may influence also on crystallization, presumably bringing about good results in the homogeneous distribution of silicon and aluminum atoms in the formed crystal.

After the preparation of aqueous reaction gel mixture in this way, the reaction mixture is maintained under the following conditions: autogenous pressure, within the range of about 120 to about 230°C for about 10 to about 50 hours.

In the crystallization process, the crystallizability can be further improved and the formation of amorphous aluminosilicate can be suppressed by adding a mineralizer to the aqueous reaction gel mixture. Such mineralizers include neutral salts of alkali or alkaline earth metals, such as NaCl, $Na_2CO_3$, $Na_2SO_4$, $Na_2SeO_4$, KCl, KBr, KF, BaCl or BaBr, among which NaCl is preferable. The amount of NaCl to be added is from about 0.1 to about 10, still preferably from about 0.1 to about 5 expressed by the molar ratio of $Cl^-/SiO_2$.

The crystalline sodium aluminosilicate of the present invention (TSZ-III) is obtained by separating the obtained crystalline aluminosilicate by filtration from the solution, washing it with water and drying it. The obtained crystals were confirmed to be a completely crystallized product by the examination of powder X-ray diffraction.

The obtained X-ray diffraction pattern is remarkably characterized in that the diffraction line of $2\theta=14.7$ ($d=6.03$ Å) is singlet and those of $2\theta=23$ ($d=3.86$ Å) and $2\theta=23.3$ ($d=3.82$ Å) are clearly split, which shows that the structure of crystalline aluminosilicate of the present invention is entirely different from that of known crystalline zeolite. As mentioned before, the arrangement of silicon and aluminum atoms on the crystal surface of the crystalline aluminosilicate of the present invention is measured by X-ray photoelectron spectroscopy, while the distribution of silicon and aluminum atoms in whole crystal is determined by the adsorption/desorption curve of pyridine, basing on the information that the aluminum atom distribution influence on the solid acid strength distribution.

In the present invention, pyridine is sufficiently adsorbed at 300°C by H (hydrogen-type)-TSZ-III obtained by the ion-exchange of crystalline aluminosilicate with ammonium chloride and/or mineral acid

and subsequent firing at about 500°C. Then the physically adsorbed pyridine is removed by maintaining it in a nitrogen stream at 300°C, and the temperature is raised at a predetermined rate to quantitatively determine the desorbed pyridine by gas chromatography or other ordinary methods of measurement. For example, when the quantitative determination was made with gas chromatograph of flame ionization detector, the peak indicates exactly the amount and distribution of the solid acid as illustrated in the examples. In this case, a sharp peak suggests the presence of solid acid sites having the similar acid strength, which indicates that the combination of silicon and aluminum bridged by an oxygen atom is comparatively homogeneous. The presence of strong solid acid sites (for example, those in which pyridine can not desorb until at around 900°C) indicate that the arrangement of aluminum atoms are coarse locally.

In order to use the crystalline sodium aluminosilicate of the present invention as catalyst or catalyst composition, the crystals are fired for dehydration at a temperature of about 200°C or above in an atmosphere of air or an inert gas. The dehydrated product is useful as catalyst or catalyst carrier for chemical reactions, and the cations in the product is preferred to be removed or replaced at least partly by heat-treatment and/or ion-exchange. Particularly those exchanged by cations are excellent as catalyst for the conversion of hydrocarbons. Usually the ions to be exchanged can be selected according to the desired reaction. They can be exchanged with hydrogen ions by acid treatment, or by exchange with $NH_4+$ followed by heat-treatment. Hydrogen ions and ions of Group VIII metals in periodical table are preferably for the decomposition, isomerization, alkylation, or other conversions of hydrocarbons.

In the present invention, the cation-exchange can be effected by contacting the crystal with a desired exchangeable cation or a cationic salt. A variety of neutral salts of Group VIII or alkaline earth metals can be used for this purpose, among which chlorides, nitrates, sulfates, and acetates are preferable.

When the TSZ-III of the present invention is used as catalyst, it is preferred to introduce active metal components by ion-exchange or to introduce hydrogen ion by acid treatment, as described above, for the improvement in catalytic activity.

The TSZ-III of the present invention can be used as a mixture with a carrier such as silica-alumina or alumina as in an ordinary method. When such an active reforming component is incorporated in the TSZ-III, raw material can be catalytically reformed under the reaction conditions of about 380 to about 500°C in temperature, about 5 to about 50 kg/cm$^2$, preferably about 10 to about 30 kg/cm$^2$ in pressure, and about 0.5 to about 5.0 V/H/V, preferably from about 1.0 to about 3.0 V/H/V in liquid space velocity.

Transition metal such as cobalt or nickel, or noble metals such as platinum or palladium can be incorporated in the TSZ-III of the present invention by ion-exchange or other methods, for the use of the TSZ-III as catalyst for various catalytic reactions. For reactions such as catalytic dewaxing of gas oil or lubricating oil fractions and catalytic isomerizations of n-paraffins, the reaction conditions are from about 250 to about 400°C in temperature, about 5 to about 50 kg/cm$^2$, preferably about 10 to about 30 kg/cm$^2$ in pressure, and about 0.25 to about 3 V/H/V, preferably about 1.0 to about 2.0 V/H/V in liquid space velocity.

For the use of TSZ-III as catalyst for catalytic conversion of alcohols into hydrocarbons, the reaction conditions are from about 250°C to about 450°C, but preferably from about 300°C to about 400°C, in temperature, from atmospheric pressure to about 50 kg/cm$^2$G, but preferably from atmospheric pressure to about 30 kg/cm$^2$G, in pressure, from about 0.1 to about 10 V/H/V, but preferably from about 0.5 to about 5 V/H/V, in liquid space velocity.

For the use of TSZ-III as catalyst for catalytic alkylation of aromatics, the reaction conditions are from about 200°C to about 550°C, but preferably from about 250°C to about 450°C, in temperature, from atmospheric pressure to about 50 kg/cm$^2$G, but preferably from about 3 kg/cm$^2$G to about 30 kg/cm$^2$G, in pressure, from about 0.1 to about 50 V/H/V, but preferably from about 1.0 to about 35 V/H/V, in liquid space velocity calculated by neglecting compounds for alkylation such as alcohols and olefins.

Moreover, for the use of the TSZ-III as catalyst for catalytic disproportionations and transalkylations of aromatic hydrocarbons, the reaction conditions are from about 300°C to about 600°C, but preferably from about 350°C to about 500°C, in temperature, from atmospheric pressure to about 50 kg/cm$^2$G, but preferably from about 3 kg/cm$^2$G to about 30 kg/cm$^2$G, in pressure, from about 0.1 to about 50 V/H/V, but preferably from about 1.0 to about 35 V/H/V, in liquid space velocity.

Examples

The following examples are provided to illustrate present invention, but are not to be construed as limiting present invention in any way.

Example 1

A solution of 12.5 g of sodium aluminate ($NaAlO_2$) in 292.5 g of water was dropped under stirring into a solution solved 269.1 g of water glass (water glass of Japanese Industrial Standard No. 3, containing 9.5% by weight of $Na_2O$ and 28.6% by weight of $SiO_2$, hereinafter abbreviated as JIS No. 3) into 135.7 g of water. Independently, a diluted hydrochloric acid was prepared by mixing 77.7 g of 35% concentrated hydrochloric acid and 175.5 g of water. These two solutions were simultaneously dropped under stirring into a solution containing 48.6 g of sodium chloride in 403.7 g of water. The final pH value of the mixture into which all solutions had been added was 8.0.

The obtained solution was charged into a 2 l autoclave and maintained under stirring for 40 hours at 185°C under autogenous pressure.

The pH value of the mother liquor after crystallization was 11.6. The crystallized product was filtered and washed after cooling, and dried overnight at 120°C.

The powder X-ray diffraction of the obtained solid product showed that the product was completely crystallized and had the lattice surfaces consistent with those of zeolite-TSZ (TSZ-III).

As the result of chemical analysis, the following chemical composition was obtained: 87.1% by weight of $SiO_2$; 5.33% by weight of $Al_2O_3$; 2.88% by weight of $Na_2O$; and 4.44% by weight of ignition loss (at 900°C). The chemical composition expressed by oxide molar ratios was

$$0.89\ Na_2O \cdot Al_2O_3 \cdot 27.8\ SiO_2 \cdot 4.7\ H_2O.$$

The product was then molded into a disc of 12 mm in diameter with a tabletting machine at a pressure of 10 ton/cm$^2$ for the the analysis of silicon and aluminum on the crystal surface. The sample was irradiated with Al-Kα ray as X-ray source at an output of 10 KV-20 mA and released X-ray photoelectrons were subjected to energy analysis to obtain an atomic ratio of silicone/aluminum of 14.5.

Consequently, the ratio of the silicon/aluminum atomic ratio on the crystal surface to the silicon/aluminum atomic ratio of the whole crystal was 1.04, from which it was concluded that the aluminum atoms were homogeneously distributed in the crystal.

Comparative Example 1

16.4 g of aluminum sulfate ($Al_2(SO_4)_3 \cdot 17.8\ H_2O$) was dissolved in a solution of 8.6 g of 95% sulfuric acid and 34.3 g of tetrapropylammonium bromide ($(C_3H_7)_4NBr$) in 105 g of water (step ①).

Separately, a solution of 154.2 g of waterglass (JIS No. 3) in 76.5 g of water was prepared (step ②).

The two solutions were simultaneously dropped under stirring into 300 g of water (step ③).

The obtained aqueous reaction gel mixture was charged into 2 l autoclave and maintained under stirring for 20 hours at 160°C under autogeneous pressure.

After the cooling of the autoclave, the crystallized product was filtered, washed, and dried overnight at 120°C. The product was further fired in an electric muffle furnace for 3 hours at 600°C, and examined by powder X-ray diffraction, showing excellent consistence in interplanar spacings of ZSM-5. The product had high crystallinity and no uncrystallized parts were observed by electron microscopy.

The results of the chemical analysis were: 89.0% by weight of $SiO_2$; 5.46% by weight of $Al_2O_3$; 1.78% by weight of $Na_2O$; and 3.41% by weight of ignition loss (at 900°C). The chemical composition expressed by oxide molar ratios, on the assumption that the ignition loss at 900°C was due to the loss of $H_2O$, was

$$0.54\ Na_2O \cdot Al_2O_3 \cdot 27.7\ SiO_2 \cdot 3.5\ H_2O.$$

The distribution of silicon and aluminum on the crystal surface was measured in the same manner as described in Example 1, obtaining the silicon/aluminum atomic ratio of 10.9. Consequently, the ratio of the silicon/aluminum atomic ratio on the crystal surface to the average silicon/aluminum atomic ratio in whole crystal was 0.79, which indicated that aluminum atoms were more distributed on the crystal surface.

Example 2

A solution of 7.5 g of sodium aluminate in 250 g of water was dropped under stirring into a solution of 234 g of waterglass (JIS No. 3) in 116 g of water. Separately, a solution of 46.7 g of 35% concentrated hydrochloric acid in 150 g of water was prepared. The two solutions were simultaneously dropped under stirring into a solution of 41.5 g of sodium chloride in 345 g of water. The final pH value of the mixture into which all solutions had been added was 11.2. The mixture was charged into a 2 l autoclave and maintained under stirring for 24 hours at 185°C under autogenous pressure.

The pH value of the mother liquor after crystallization was 12.0. The crystallized product was filtered and washed after cooling, then dried overnight at 120°C.

The powder X-ray diffraction of the obtained solid product showed that the product was completely crystallized and consistent with TSZ-zeolite in interplanar spacing.

The results of the chemical analysis were: 88.3% by weight of $SiO_2$; 3.82% by weight of $Al_2O_3$; 2.52% by weight of $Na_2O$; and 7.34% by weight of ignition loss. The chemical composition expressed in terms of oxide molar ratios was

$$1.1\ Na_2O \cdot Al_2O_3 \cdot 39.3\ SiO_2 \cdot 10.9\ H_2O.$$

The atomic ratio of silicon to aluminum on the crystal surface determined by X-ray photoelectron spectroscopy in the same manner as described in Example 1 was 23.6.

Consequently, the ratio of the silicon/aluminum atomic ratio on the crystal surface to the average silicon/aluminum atomic ratio in whole crystal was 1.20, which indicated that the aluminum atoms were more distributed inside than on the surface of the crystal.

Example 3

A solution of 5.4 g of sodium aluminate in 292.5 g of water was dropped under stirring into a solution of

201.2 g of colloidal silica (Ludox-40®), containing 20% by weight of $SiO_2$, in 135.7 g of water. Separately, a solution of 58.1 g of 35% concentrated hydrochloric acid in 175.5 g of water was prepared.

The two solutions were simultaneously dropped under stirring into a solution of 48.8 g of sodium chloride in 403.7 g of water. The final pH value of the mixture into which all solutions had been added was 10.4. The obtained aqueous reaction gel mixture was charged in a 2 l autoclave and maintained under stirring for 24 hours at 185°C under autogenous pressure.

The pH value of the mother liquor after crystallization was 12 or more. The crystallized product was filtered and washed after cooling, then dried overnight at 120°C.

The powder X-ray diffraction of the obtained solid product showed that the product was completely crystallized and consistent with TSZ-zeolite in interplanar spacing.

The results of the chemical analysis were: 88.7% by weight of $SiO_2$; 2.35% by weight of $Al_2O_3$; 1.64% by weight of $Na_2O$; and 6.40% by weight of ignition loss. The chemical composition expressed by oxide molar ratio was $1.15\ Na_2O \cdot Al_2O_3 \cdot 64.2\ SiO_2 \cdot 15.4\ H_2O$.

The atomic ratio of silicon to aluminum on the crystal surface determined by X-ray photoelectron spectroscopy in the same manner as described in Example 1 was 35.0.

Consequently, the ratio of the silicon/aluminum atomic ratio on the crystal surface to the average silicon/aluminum atomic ratio in whole crystal was 1.09.

Example 4

A solution of 5.4 g of sodium aluminate in 293.0 g of water was dropped under stirring into a solution of 269.1 g of water-glass (JIS No. 3) in 135.7 g of water. Separately a solution of 70.6 g of 35% concentrated hydrochloric acid in 175.5 g of water was prepared.

The two solutions were simultaneously dropped under stirring into a solution of 48.8 g of sodium chloride in 403.7 g of water. The final pH value of the mixture into which all solutions had been added was 8.8. The obtained aqueous reaction gel mixture was charged in a 2 l autoclave and maintained under stirring for 24 hours at 186°C under autogenous pressure.

The pH value of the mother liquor after crystallization was 12.0. The crystallized product was filtered and washed after cooling, then dried overnight at 120°C.

The powder X-ray diffraction of the obtained solid product showed that the product was completely crystallized and consistent with TSZ-zeolite in interplanar spacing.

The results of the chemical analysis were: 86.0% by weight of $SiO_2$; 2.57% by weight of $Al_2O_3$; 2.44% by weight of $Na_2O$; and 6.40% by weight of ignition loss. The chemical composition expressed by oxide molar ratio was

$$1.56\ Na_2O \cdot Al_2O_3 \cdot 56.9\ SiO_2 \cdot 19.0\ H_2O.$$

The silicon/aluminum ratio on the crystal surface of the obtained sample, measured in the same manner as described in Example 1, was the same as the silicon/aluminum ratio calculated from the above chemical analysis values.

75 ml of an aqueous solution of 5% by weight of ammonium chloride was added to 5 g of the obtained crystalline sodium aluminosilicate to effect ion-exchange at 80°C for 1.5 hours. The ion-exchange treatments were carried out four times, and then the sample was washed with water, dried at 110°C, and calcined in air at 550°C for 3 hours to form H-TSZ-III (hydrogen-type). The $Na_2O$ content in the H-TSZ-III was not more than 0.01% by weight.

Comparative Examples 2, 3 and 4

Crystalline aluminosilicates having a variety of silicon/aluminum atomic ratios as shown in Table 3 were prepared in the same manner as described in Comparative Example 1.

TABLE 3

| Step | Solution to be mixed | Comparative Example 2 | Comparative Example 3 | Comparative Example 4 |
|---|---|---|---|---|
| ① | aluminum sulfate | 3.0 | 3.9 | 5.5 |
| | water | 140 | 140 | 140 |
| | tetrapropylammonium bromide | 45.7 | 45.7 | 45.7 |
| ② | water-glass | 207 | 207 | 207 |
| | water | 102 | 102 | 102 |
| ③ | water | 400 | 400 | 400 |

The value in the table are expressed by gram unit.

These aqueous gel mixtures were maintained at 160°C for 20 hours and subjected to powder X-ray diffraction in the same manner as described in Comparative Example 1. All the samples showed the diffraction peaks of ZSM-5 with good crystallinity. The results of the chemical analysis of the samples after calcining at 600°C for 3 hours are summarized in Table 4.

TABLE 4

| | Comparative Example 2 | Comparative Example 3 | Comparative Example 4 |
|---|---|---|---|
| $Na_2O$ | 0.68 | 0.81 | 0.98 |
| $SiO_2$ | 87.4 | 87.0 | 86.4 |
| $Al_2O_3$ | 1.18 | 1.57 | 2.06 |
| Ignition loss | 11.5 | 10.1 | 9.98 |
| $SiO_2/Al_2O_3$ | 125.4 | 93.8 | 71.2 |
| $Na_2O/Al_2O_3$ | 0.95 | 0.85 | 0.78 |

The values in Table 4 are expressed by weight % except the ratios expressed by molar ratio.

The silicon and aluminum on the crystal surface of the obtained ZSM-5 was analyzed in the same manner as described in Example 1, the results of which are summarized in Table 5.

Comparative Example 5

7.0 g of aluminum sulfate was dissolved in a solution of 13.0 g of 95% sulfuric acid and 34.3 g of tetrapropylammonium bromide in 105 g of water.

Separately, a solution of 154.2 g of water-glass (JIS No. 3) in 76.5 g of water was prepared. These two solutions were dropped into 300 g of water to obtain an aqueous reaction mixture gel. ZSM-5 was obtained by crystallizing said gel in the same manner as described in Comparative Example 1. The obtained ZSM-5 was partly calcined at 600°C for 3 hours and subjected to chemical analysis, the result of which was: 94.5% by weight of $SiO_2$; 2.57% by weight of $Al_2O_3$; 1.22% by weight of $Na_2O$; and 1.76% by weight of ignition loss. The chemical composition expressed by oxide molar ratio was

$$0.78\ Na_2O \cdot Al_2O_3 \cdot 62.5\ SiO_2 \cdot 3.9\ H_2O.$$

The obtained ZSM-5, fired at 600°C for 3 hours, was ion-exchanged in the same manner as described in Example 4 to prepare H (hydrogen type)-ZSM-5, the $Na_2O$ content of which was 0.01% by weight or less.

TABLE 5

| | Ratio | Comparative Example 2 | Comparative Example 3 | Comparative Example 4 |
|---|---|---|---|---|
| I | Silicon/aluminum atomic ratio (X-ray photoelectron method) | 52.0 | 41.7 | 31.3 |
| II | Silicon/aluminum atomic ratio (chemical analysis) | 62.7 | 46.9 | 35.6 |
| | I/II | 0.83 | 0.89 | 0.88 |

Example 5

The hydrogen-type TSZ-III and the hydrogen-type ZSM-5 obtained in Example 4 and Comparative Example 5 were subjected to pyridine adsorption and heat desorption test.

In order to prevent the changes in pressure when pyridine is adsorbed or desorbed, the sample was made into granules of 30/100 mesh by molding with an alumina binder was prepared as follows. (The units of mesh used in the following are to be understood as Tyler mesh size designating the number of holes per 2.54 cm).

20.2 g of sodium aluminate was dissolved in 26.4 g of water, and 28.8 g of aluminum sulfate was dissolved in 50.7 g of water. These two solutions were simultaneously dropped into 480 g of hot water heated at 70 to 80°C to prepare gel. The obtained gel was aged at 70°C for about 1 hour and filtered. The

product was then washed with an aqueous solution of 1.5% by weight of ammonium carbonate until the $Na_2O$ content after firing at 600°C reached 50 ppm or less in terms of weight.

The obtained gel was kneaded and molded so that the binder (alumina) content was 30% by weight. The sample was prepared by drying the molded gel at 120°C, calcining it at 600°C for 3 hours, and granulating it into 30/100 mesh granules.

0.075 g of the sample, accurately weighed, was charged into an adsorption tube and dried at 600°C for 1 hour in a dry nitrogen stream. Nitrogen was flown into the pyridine solution at a constant temperature of 15.5°C at a flow velocity of 50 ml/min. so that pyridine was adsorbed by the sample of 300°C to saturation. Then only dry nitrogen was flown at a maintained temperature of 300°C to remove the physically adsorbed pyridine.

The sample was then heated at a rate of 10°C/min and the desorbed pyridine was quantitatively determined by flame ionization detecting gas chromatography. The temperature-pyridine desorption relationships of the samples of Example 4 and Comparative Example 5 are shown in Figure 1.

The samples obtained in Example 1 and Comparative Example 1 were then ion-exchanged in the same manner as described in Example 4 to prepare hydrogen-type TSZ-III and hydrogen-type ZSM-5. These products were made into 30/100 mesh granules in the same manner as described in Example 5 (the present example) and subjected to the same pyridine adsorption and desorption test. The results are shown in Figure 2.

It is confirmed from Figures 1 and 2 that the sample of the present invention (TSZ-III) shows more sharp distribution than ZSM-5, which indicate that TSZ-III has relatively larger amount active sites with the same acid strength than ZSM-5 has. As the result of the comparison on the silica/alumina ratio of the same level, the amount of adsorbed pyridine of TSZ-III is larger than that of ZSM-5.

The amounts of the adsorbed pyridine are as follows:

| The present invention | | SZM-5 | |
|---|---|---|---|
| Example 1 | Example 4 | Comp. Ex. 1 | Comp. Ex. 5 |
| 0.346 | 0.192 | 0.188 | 0.146 |

(unit: m moleq/g)

Example 6

Toluene was alkylated with ethylene using the hydrogen-type TSZ-III or the hydrogen-type ZSM-5, (which had been prepared in Example 5 using TSZ-III obtained in Example 1 and ZSM-5 obtained in Comparative Example 1 respectively), as catalyst, under the following conditions.

An atmospheric flow reactor was filled with 2.0 ml of a 25/60 mesh granule catalyst, into which toluene, ethylene and hydrogen were introduced in a molar ratio of 5/1/5/at a reaction temperature of 350°C at a space velocity (V/H/V) of 3.7/340/1,680. The proportions of ethylene and hydrogen were determined based on the volumes of these gases at 20°C. p-, m-, and o-ethyltoluenes were produced by the above reaction, among which p-ethyltoluene is of industrial value as material for synthetic resin because it can afford p-methylstyrene by the dehydrogenation of the ethyl group.

The results of the analysis of the products by gas chromatography were as follows:

| | Toluene conversion | p-Ethyltoluene selectivity |
|---|---|---|
| H-ZSM-5 (Comp. Ex. 1) | 19.74 | 27.9 |
| H-TSZ-III (Ex. 1) | 18.82 | 33.1 |

It was confirmed by the observation of the electron microscopy that the crystals of the hydrogen-type TSZ-III had almost the same size, approximately 0.5×1.0 µm, as those of the hydrogen-type ZSM-5 before they were molded with a binder. Therefore, results of present example illustrate that the p-Ethyltoluene selectivity of TSZ-III is better than that of ZSM-5.

Example 7

The crystalline aluminosilicate obtained in Example 3 was converted into a hydrogen-type by the method described in Example 4, and kneaded with a binder by the method described in Example 5, to mold into a pellet of 1.5 mm in diameter. The ZSM-5 obtained in Comparative Example 4 was also converted into a hydrogen-type by the method of Example 4 and kneaded with a binder by the method of Example 5 to mold into a pellet of 1.5 mm in diameter. Both of the pellets were prepared so that the amount of the binder, after firing at 600°C for 3 hours, was 30% by weight.

Toluene was alkylated with methanol, using the obtained catalysts, and the resulting xylenes were

examined. The reactions were effected using 2.0 ml of the catalysts (25/60 mesh) in an atmospheric flow reactor, and the products were analyzed by gas chromatography.

As understood from the results shown in Table 6, the superiority of the catalyst of the present invnetion in the toluene conversion, the yield of $C_8$ aromatics, and the selectivity of p-xylene was proved.

Example 8

The crystalline aluminosilicate obtained in Example 2 was converted into a hydrogen-type by the method described in Example 4, and kneaded with a binder by the method described in Example 5 to mold into a pellet of 1.5 mm in diameter. The pellet was prepared so that the amount of the binder was 30% by weight after calcining at 600°C for 3 hours.

The hydrogen-type ZSM-5 was prepared as follows for the comparison in the present example: 11.6 g of aluminum sulfate was dissolved in a solution of 10.8 g of 95% sulfuric acid and 34.3 g of tetrapropylammonium bromide in 105 g of water. Separately, a solution of 154.2 g of water-glass (JIS No. 3) in 76.5 g of water was prepared. These two solutions were dropped into 300 g of water to obtain an aqueous reaction mixture gel. The gel was crystallized in the manner as described in Comparative Example 1 to obtain ZSM-5. The result of the chemical analysis of the product after calcining at 600°C for 3 hours was: 91.4% by weight of $SiO_2$; 4.11% by weight of $Al_2O_3$; 1.77% by weight of $Na_2O$; and 2.76% by weight of ignition loss. The chemical composition expressed in oxide molar ratio was

$$0.71\ Na_2O \cdot Al_2O_3 \cdot 37.8\ SiO_2 \cdot 3.8\ H_2O.$$

The obtained ZSM-5 was converted into a hydrogen-type by the method described in Example 4, and kneaded with a binder by the method described in Example 5, to mold into a pellet of 1.5 mm in diameter. The pellet was prepared so that the amount of the binder was 30% by weight after calcining at 600°C for 3 hours.

TABLE 6

| Crystalline aluminosilicate | | Example 3 | Comparative Example 4 |
|---|---|---|---|
| Toluene conversion (%) | | 26.6 | 24.6 |
| Aromatic product yield (% based on the amount of toluene) | | 100.3 | 100.8 |
| Aromatic product distribution (%) | $C_6$ component | 2.2 | 5.7 |
| | $C_8$ component | 81.2 | 71.7 |
| | $C_9$ component | 15.4 | 21.5 |
| | $C_{10}$ component | 1.2 | 1.1 |
| $C_8$ aromatic isomer distribution (%) | ethylbenzene | 0.8 | 1.8 |
| | p-xylene | 33.1 | 23.7 |
| | m-xylene | 46.9 | 52.8 |
| | o-xylene | 19.2 | 21.7 |

Reaction conditions:
- Raw material: toluene/methanol=2/1 (mol/mol)
- Treating gas: hydrogen/toluene=4/1 (mol/mol)
- Reaction temperature: 400°C
- Reaction pressure: atmospheric
- Space velocity: 5.6 V/H/V based on the raw material

Toluene was disproportionated in the presence of the obtained catalysts and the resulting benzene and xylenes were examined. The reactions were effected with 1.0 ml of the catalysts (25/60 mesh) in an atmospheric flow reactor, and the products were analyzed by gas chromatography.

As understood from the results shown in Table 7, the superiority of the catalyst of the present invention in the yield of $C_8$ aromatics and the selectivity of p-xylene was proved.

TABLE 7

| Crystalline aluminosilicate | TSZ | ZSM-5 |
|---|---|---|
| Toluene conversion (%) | 10.8 | 11.2 |
| Aromatic product yield (%) (based on the amount of toluene, mol%) | 99.0 | 98.7 |
| Aromatic product distribution (mol%) | | |
| $C_6$ | 49.7 | 55.1 |
| $C_8$ | 48.7 | 43.4 |
| $C_9$+ | 1.6 | 1.5 |
| $C_8$ Aromatic isomer distribution (%) | | |
| ethylbenzene | less than 0.1 | less than 0.1 |
| p-xylene | 29.7 | 23.9 |
| m-xylene | 49.2 | 52.9 |
| o-xylene | 21.1 | 23.2 |

Reaction conditions:
Raw material: toluene
Treating gas: hydrogen/toluene=4/1 (mol/mol)
Reaction temperature: 420°C
Reaction pressure: atmospheric
Space velocity: 14.8 V/H/V based on toluene

Example 9

Catalysts containing nickel (Ni) were prepared using H-TSZ III which was made in Example 1 and H-ZSM-5 which was made in Comparative Example 1. The methods to introduce Ni in both H-TSZ III and H-ZSM-5 were same — H-TSZ III and H-ZSM-5 were treated with 1 normal $Ni(NO_3)_2$ for 1 hour at 80°C, washed with water, dried then calcined for 3 hours at 600°C. Amounts of nickel contained in H-TSZ III and H-ZSM-5 were 0.65 and 0.68 weight % respectively. Using above obtained Ni containing catalysts, catalytic dewaxing of gas oil were carried out under reaction conditions written in Table 8.

TABLE 8

| Condition | Ni/H-TSZ-III | | Ni/H-ZSM-5 | |
|---|---|---|---|---|
| Temperature (°C) | 310 | 370 | 315 | 380 |
| LHSV (V/H/V) | 2 | 2 | 2 | 2 |
| Pressure ($kg/cm^2G$) | 42 | 42 | 42 | 42 |
| Treat gas rate ($l-H_2/l$-feed) | 450 | 450 | 450 | 450 |

The characteristics of gas oil were as follows:
specific gravity (15/4°C): 0.8753
sulfur content (weight %): 1.96
Nitrogen content (weight %): 0.026
Ratio of carbon/hydrogen by weight: 6.69
Pour point (°C): 15
Distillation test (ASTM D-2887), °C

| | |
|---|---|
| initial point: | 177°C |
| 5%: | 279 |
| 10%: | 306 |
| 30%: | 347 |
| 50%: | 371 |
| 70%: | 394 |
| 90%: | 425 |
| 95%: | 440 |
| 97%: | 450 |

The results were as follows:

| Product oil | Ni/H-TSZ-III | | Ni/H-ZSM-5 | |
|---|---|---|---|---|
| Yield (weight % on feed) | 74 | 62 | 71 | 60 |
| Pour point (°C) | −15 | −40 | −15 | −40 |

These results illustrate that the catalyst obtained in this invention is excellent.

## Claims

1. A crystalline aluminosilicate, characterized in that the value of the silicon/aluminum atomic ratio of the external surface of the crystal is not smaller than the average value of the silicon/aluminum atomic ratio in the whole crystal.

2. A crystalline aluminosilicate as set forth in Claim 1, characterized by comprising a salt prepared with one or more ions selected from $H^+$, $Na^+$, $NH_4^+$ and cations of a Group VIII metal in the periodic table.

3. A crystalline sodium aluminosilicate as set forth in Claim 1 or 2, characterized by having the following chemical composition expressed in terms of oxide molar ratios:

$$0.8{\sim}1.6\ Na_2O \cdot Al_2O_3 \cdot 10{\sim}100\ SiO_2 \cdot 0{\sim}40\ H_2O$$

and a powder X-ray diffraction pattern showing at least the following interplanar spacings:

| Interplanar spacing (Å) | Relative intensity ($I/I_o$) |
|---|---|
| 11.2 ±0.2 | strong |
| 10.1 ±0.2 | strong |
| 3.86±0.05 | very strong |
| 3.72±0.05 | strong |
| 3.64±0.05 | strong |

4. A process for the preparation of a crystalline sodium aluminosilicate, by making a homogeneous aqueous reaction mixture of silicon and aluminum compounds using sodium aluminate and water glass, said aqueous reaction mixture substantially consisting of inorganic reaction materials and having the following composition expressed in terms of molar ratios:

| | |
|---|---|
| $SiO_2/Al_2O_3$ | 10~100 |
| $Na_2O/SiO_2$ | 0.03~0.5 |
| $H_2O/SiO_2$ | 10~300 |
| $Cl^-/SiO_2$ | 0.1~10 |

gelling said reaction mixture with a mineral acid and continuously heating and maintaining the gelled reaction mixture at a crystallization temperature until crystals form.

5. A process for the preparation of a sodium aluminosilicate as set forth in claim 4, characterized in that said aqueous reaction mixture has the following composition expressed in terms of molar ratios:

| | |
|---|---|
| $SiO_2/Al_2O_3$ | 20~80 |
| $Na_2O/SiO_2$ | 0.05~0.3 |
| $H_2O/SiO_2$ | 20~200 |
| $Cl^-/SiO_2$ | 0.1~5 |

6. A process for the preparation of a crystalline aluminosilicate, characterized by that crystalline sodium aluminosilicate wherein the value of the silicon/aluminum atomic ratio on the external surface of the crystal is not smaller than the average value of the silicon/aluminum atomic ratio in whole crystal, having the following chemical composition expressed in terms of oxide molar ratios:

$$0.8\sim1.6\ Na_2O \cdot Al_2O_3 \cdot 10\sim100\ SiO_2 \cdot 0\sim40\ H_2O$$

and showing at least the following interplanar spacings observed by powder X-ray diffraction method:

| Interplanar spacing (Å) | Relative intensity ($I/I_o$) |
|---|---|
| 11.2 ±0.2 | strong |
| 10.1 ±0.2 | strong |
| 3.86±0.05 | very strong |
| 3.72±0.05 | strong |
| 3.64±0.05 | strong |

is brought into contact with a desired cation or a cationic salt, to replace at least a part of the sodium ions of said crystalline sodium aluminosilicate with other cations.

7. A process for the preparation of a hydrogen ion-exchanged crystalline aluminosilicate as set forth in Claim 6, characterized by contacting the crystalline sodium aluminosilicate with an acid.

8. A process for the preparation of an ammonium ion-exchanged crystalline aluminosilicate as set forth in Claim 6, characterized by replacing at least a part of the sodium ions of the crystalline sodium aluminosilicate with $NH_4^+$.

9. A process for the preparation of a metallic ion-exchanged crystalline aluminosilicate as set forth in Claim 6, characterized by replacing at least a part of the sodium ions of the crystalline sodium aluminosilicate with ions of Group VIII metal in the periodic table.

10. A process for the conversion of an organic material, characterized by using calcined crystalline aluminosilicate as catalyst; wherein cation include one or more ions selected from $H^+$, $Na^+$, $NH_4^+$ and cations of Group VIII metal in the periodic table, the value of the silicon/aluminum atomic ratio of the external surface of said crystal is substantially not smaller than average value of the silicon/aluminum atomic ratio in the whole crystal; under the conversion conditions of organic materials.

**Patentansprüche**

1. Ein kristallines Aluminiumsilicat, dadurch gekennzeichnet, daß der Wert des Silizium/Aluminium-Atomverhältnisses der äußeren Kristalloberfläche nicht geringer ist als der durchschnittliche Wert des Silizium/Aluminium-Atomverhältnisses in dem ganzen Kristall.

2. Ein kristallines Aluminiumsilicat nach Anspruch 1, dadurch gekennzeichnet, daß es ein mit einem oder mehreren Ionen aus der Gruppe $H^+$, $Na^+$, $NH_4^+$ und Kationen eines Metalls der Gruppe VIII in der Tafel des periodischen Systems präpariertes Salz umfaßt.

3. Ein kristallines Natriumaluminiumsilicat nach Anspruch 1 oder 2, dadurch gekennzeichnet, daß es die folgende chemische Zusammenzetzung ausgedrückt in der Form von Oxidmolverhältnissen aufweist:

$$0{,}8\sim1{,}6\ Na_2O \cdot Al_2O_3 \cdot 10\sim100\ SiO_2 \cdot 0\sim40\ H_2O$$

besitzt und ein Pulverröntgenbeugungsbild mit mindestens den folgenden netzebenen Abständen aufweist:

| Netzebenenabstand (Å) | Relative Intensität ($I/I_o$) |
|---|---|
| 11,2 ±0,2 | stark |
| 10,1 ±0,2 | stark |
| 3,86±0,05 | sehr stark |
| 3,72±0,05 | stark |
| 3,64±0,05 | stark |

4. Ein Verfahren zur Herstellung eines kristallinen Natriumaluminiumsilicats durch Anfertigung eines homogenen wässerigen Reaktionsgemisches von Silizium- und Aluminiumverbindungen unter Einsatz von Natriumaluminat und Wasserglas, wobei das besagte wässerige Reaktionsgemisch im wesentlichen aus anorganischen Reaktionsstoffen besteht und die folgende in der Form von Molverhältnissen ausgedrückte Zusammensetzung besitzt:

| | |
|---|---|
| $SiO_2/Al_2O_3$ | 10~100 |
| $Na_2O/SiO_2$ | 0,03~0,5 |
| $H_2O/SiO_2$ | 10~300 |
| $Cl^-/SiO_2$ | 0,1~10 |

worauf das besagte Reaktionsgemisch mit einer Mineralsäure geliert und kontinuierlich erhitzt wird und das gelierte Reaktionsgemisch bei Kristallisationstemperatur gehalten wird, bis sich Kristalle bilden.

5. Ein Verfahren zur Herstellung eines Natriumaluminiumsilicats nach Anspruch 4, dadurch gekennzeichnet, daß das besagte wässerige Reaktionsgemisch die folgende in der Form von Molverhältnissen ausgedrückte Zusammensetzung besitzt:

| | |
|---|---|
| $SiO_2/Al_2O_3$ | 20~80 |
| $Na_2O/SiO_2$ | 0,05~0,3 |
| $H_2O/SiO_2$ | 20~200 |
| $Cl^-/SiO_2$ | 0,1~5 |

6. Ein Verfahren zur Herstellung eines kristallinen Aluminiumsilicats, dadurch gekennzeichnet, daß kristallines Natriumaluminiumsilicat, bei dem der Wert des Silizium/Aluminium-Atomverhältnisses an der äußeren Kristalloberfläche nicht geringer ist als der durchschnittliche Wert des Silizium/Aluminium-Atomverhältnisses in dem ganzen Kristall und das die folgende chemische Zusammensetzung ausgedrückt in der Form von Oxidmolverhältnissen

$$0{,}8\sim1{,}6\ Na_2O \cdot Al_2O_3 \cdot 10\sim100\ SiO_2 \cdot 0\sim40\ H_2O$$

besitzt und mindestens die folgenden nach dem Pulverröntgenbeugungsverfahren beobachteten Netzebenenabstände:

| Netzebenenabstand (Å) | Relative Intensität ($I/I_o$) |
|---|---|
| 11,2 ±0,2 | stark |
| 10,1 ±0,2 | stark |
| 3,86±0,05 | sehr stark |
| 3.72±0,05 | stark |
| 3,64±0,05 | stark |

aufweist, mit einem gewünschten Kation oder einem kationischen Salz in Kontakt gebracht wird, um mindestens einen Teil der Natriumionen des besagten kristallinen Natriumaluminiumsilicats durch andere Kationen zu ersetzen.

7. Ein Verfahren zur Herstellung eines wasserstoffionenausgetauschten kristallinen Aluminiumsilicats nach Anspruch 6, dadurch gekennzeichnet, daß das kristalline Natriumaluminiumsilicat mit einer Säure in Kontakt gebracht wird.

8. Ein Verfahren zur Herstellung eines ammoniumionenausgetauschten kristallinen Aluminiumsilicats nach Anspruch 6, dadurch gekennzeichnet, daß mindestens ein Teil der Natriumionen des kristallinen Natriumaluminiumsilicats durch $NH_4^+$ ersetzt wird.

9. Ein Verfahren zur Herstellung eines metallionenausgetauschten kristallinen Aluminiumsilicats nach Anspruch 6, dadurch gekennzeichnet, daß mindestens ein Teil der Natriumionen des kristallinen Natriumaluminiumsilicats durch Ionen eines Metalls der Gruppe VIII in der Tafel des periodischen Systems ersetzt wird.

10. Ein Verfahren für die Umformung eines organischen Stoffes, dadurch gekennzeichnet, daß von einem gebrannten kristallinen Aluminiumsilicat als Katalysator Gebrauch gemacht wird, in dem zu den Kationen ein oder mehr Ionen aus der Gruppe $H^+$, $Na^+$, $NH_4^+$ sowie Kationen aus Metallgruppe VIII in der Tafel des periodischen Systems zählen, der Wert des Silizium/Aluminium-Atomverhältnisses der äußeren Fläche des besagten Kristalls im wesentlichen nicht geringer ist als der durchschnittliche Wert des Silizium/Aluminium-Atomverhältnisses in dem ganzen Kristall, und zwar unter den Umsetzbedingungen organischer Stoffe.

**Revendications**

1. Un aluminosilicate cristallin caractérisé en ce que la valeur du rapport atomique silicium/aluminium à la surface externe du cristal n'est pas inférieure à la valeur moyenne du rapport atomique silicium/aluminium dans le cristal tout entier.

2. Un aluminosilicate cristallin comme décrit dans la revendication 1 caractérisé en ce qu'il comprend un sel préparé avec un ou plusieurs ions choisis parmi $H^+$, $Na^+$, $NH_4^+$ et les cations des métaux du Groupe VIII du tableau périodique des éléments.

3. Un aluminosilicate de sodium cristallin comme décrit dans les revendications 1 ou 2 caractérisé en ce qu'il présente la composition chimique suivante exprimée en fonction des rapports molaires des oxydes:

$$0{,}8\sim1{,}6\ Na_2O \cdot Al_2O_3 \cdot 10\sim100\ SiO_2 \cdot 0\sim40\ H_2O$$

et un schéma de diffraction des rayons X par la méthode des poudres indiquant au moins les écartements des faces suivants:

| Ecartements des plans (Å) | intensité relative ($I/I_o$) |
|---|---|
| 11,2 ±0,2 | forte |
| 10,1 ±0,2 | forte |
| 3,86±0,05 | très forte |
| 3,72±0,05 | forte |
| 3,64±0,05 | forte |

4. Un procédé pour préparer un aluminosilicate de sodium cristallin en formant un mélange réactionnel aqueux homogène de composés de silicium et d'aluminium utilisant l'aluminate de sodium et le verre soluble, ledit mélange réactionnel aqueux comportant substantiellement des matières réactives inorganiques et possédant la composition suivante, exprimée en fonction des rapports molaires:

| | |
|---|---|
| $SiO_2/Al_2O_3$ | 10~100 |
| $Na_2O/SiO_2$ | 0,03~0,5 |
| $H_2O/SiO_2$ | 10~300 |
| $Cl^-/SiO_2$ | 0,1~10 |

faisant gélifier ledit mélange avec un acide minéral, et chauffant continûment et maintenant le mélange réactionnel gélifié à une température de cristallisation jusqu'à ce que des cristaux se forment.

5. Un procédé pour la préparation d'un aluminosilicate de sodium comme décrit dans la revendication 4 caractérisé en ce que le mélange réactionnel aqueux possède la composition suivante, exprimée en fonction des rapports molaires:

| | |
|---|---|
| $SiO_2/Al_2O_3$ | 20~80 |
| $Na_2O/SiO_2$ | 0,05~0,3 |
| $H_2O/SiO_2$ | 20~200 |
| $Cl^-/SiO_2$ | 0,1~5 |

6. Un procédé pour la préparation d'un aluminosilicate de sodium cristallin, caractérisé en ce que cet aluminosilicate de sodium cristallin où la valeur du rapport atomique silicium/aluminium à la surface externe du cristal n'est pas inférieure à la valeur moyenne du rapport atomique silicium/aluminium dans le cristal tout entier, possédant la composition chimique suivante exprimée en fonction des rapports molaires des oxydes:

$$0{,}8\sim1{,}6\ Na_2O \cdot Al_2O_3 \cdot 10\sim100\ SiO_2 \cdot 0\sim40\ H_2O$$

et présentant au moins les écartements des plans suivants lorsqu'observés par diffraction des rayons X par la méthode des poudres:

| Ecartements des plans (Å) | intensité relative ($I/I_o$) |
|---|---|
| 11,2 ±0,2 | forte |
| 10,1 ±0,2 | forte |
| 3,86±0,05 | très forte |
| 3,72±0,05 | forte |
| 3,64±0,05 | forte |

est mis en contact avec un cation désiré ou un sel cationique, pour remplacer au moins une partie des ions sodiques dudit aluminosilicate de sodium cristallin par d'autres cations.

7. Un procédé pour la préparation d'un aluminosilicate cristallin dont au moins une partie des cations ont été remplacés par des ions d'hydrogène comme décrit dans la revendication 6, caractérisé en ce que l'aluminosilicate de sodium cristallin est mis en contact avec un acide.

8. Un procédé pour la préparation d'un aluminosilicate cristallin dont au moins une partie des cations ont été remplacés par des ions ammoniques comme décrit dans la revendication 6, caractérisé en ce qu'au moins une partie des ions sodiques de l'aluminosilicate de sodium cristallin sont remplacés par $NH_4^+$.

9. Un procédé pour la préparation d'un aluminosilicate cristallin dont au moins une partie des cations ont été remplacés par des ions métalliques, comme décrit dans la revendication 6, caractérisé en ce qu'au moins une partie des ions sodiques de l'aluminosilicate de sodium cristallin sont remplacés par les ions d'un métal du Groupe VIII du tableau périodique des éléments.

10. Un procédé pour la transformation d'une matière organique, caractérisé en ce qu'on utilise comme catalyseur de l'aluminosilicate cristallin calciné; où les cations comprennent un ou plusieurs ions choisis parmi $H^+$, $Na^+$, $NH_4^+$ et les cations des métaux du Groupe VIII du tableau périodique, la valeur du rapport atomique silicium/aluminium à la surface externe dudit cristal n'est substantiellement pas inférieure à la valeur moyenne du rapport atomique silicium/aluminium dans le cristal tout entier; dans les conditions requises pour la transformation de matières organiques.

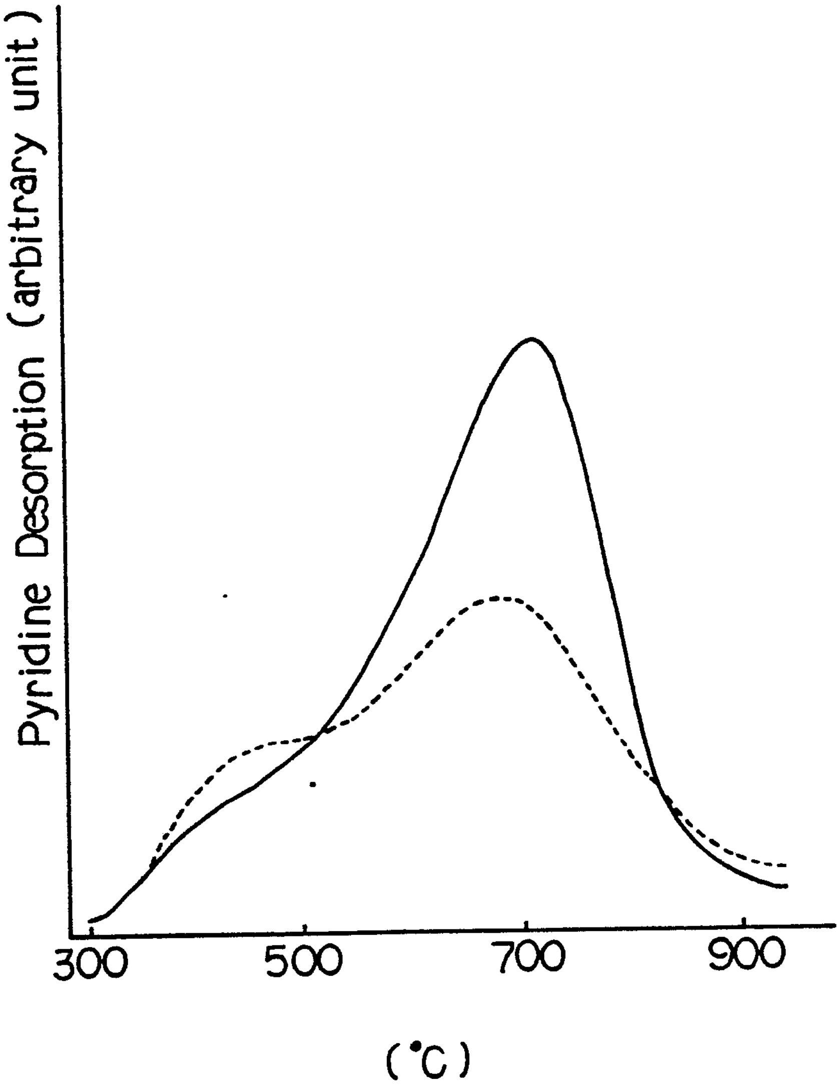


FIG. 1

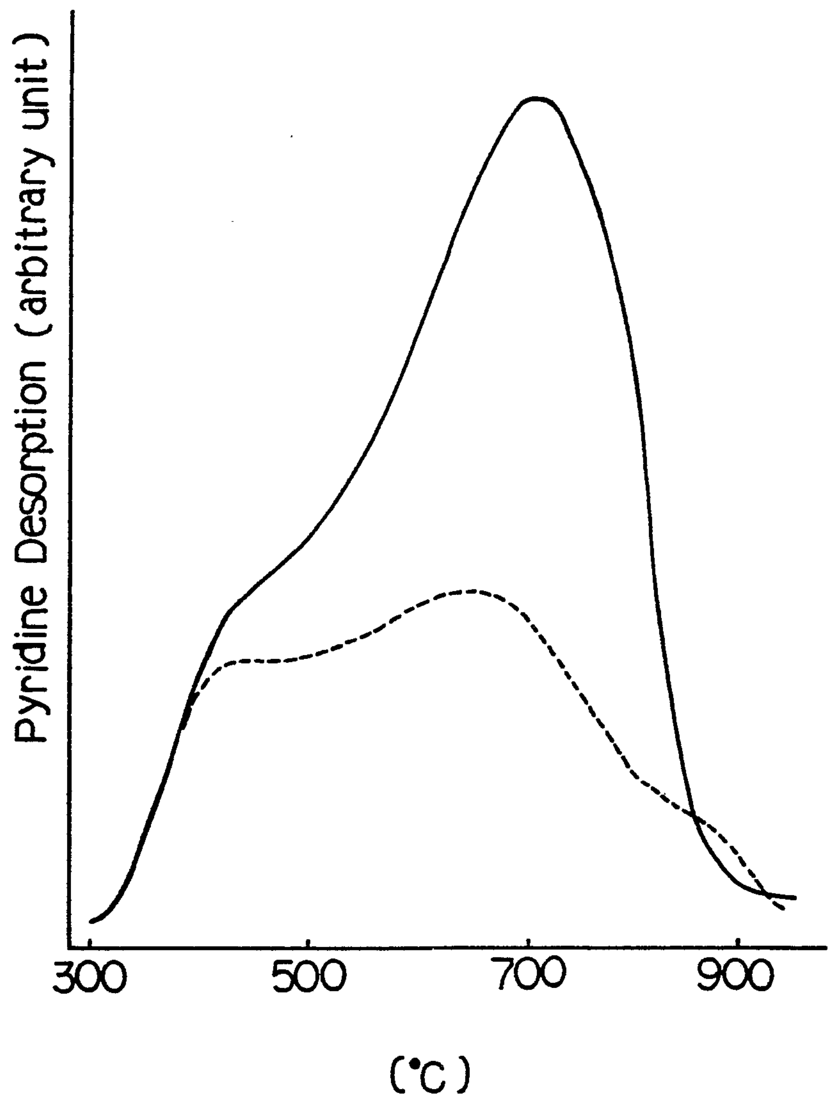


FIG. 2